# EUROPEAN PATENT SPECIFICATION

(11) **EP 2 185 121 B1**
(45) Date of publication and mention of the grant of the patent: **21.09.2011**
(21) Application number: 08784469.2
(22) Date of filing: 22.08.2008
(51) Int. Cl.: A61F 17/00, A61B 17/50

(54) **DEVICE FOR TREATING STINGS FROM STINGING ORGANISMS**
VORRICHTUNG ZUR BEHANDLUNG VON STICHEN VON STECHENDEN ORGANISMEN
DISPOSITIF POUR TRAITER DES PIQÛRES D'ORGANISMES PIQUEURS

(30) Priority: 22.08.2007 DK 200701201
(43) Date of publication of application: 19.05.2010
(73) Proprietor: Skancard Aps, 8700 Horsens (DK)
(72) Inventor: HANSEN, Per, Kirkegaard, DK-8660 Skanderborg (DK)
(74) Representative: Henriksen, Michael
(86) International application number: PCT/DK2008/050211
(87) International publication number: WO 2009/024157

(56) References cited:
- WO-A-03/022094
- DE-U1-202006 010 549
- FR-A- 2 791 326
- US-A1- 2006 271 069

## Description

### FIELD OF THE INVENTION

The invention relates to treating a sting of stinging organisms after having been stung. The invention relates to providing agents for inhibiting or alleviating the sourness and illness of having been stung, and in relation to some very dangerous species of coelenterates, the invention relates to agents for surviving after having been stung.

### BACKGROUND OF THE INVENTION

Jellyfish are part of an aquatic invertebrate phylum known as coelenterates. This group of creatures is divided into four different classes: hydrozoa (fire coral), scyphozoa (sea nettle), cubozoa (box jellyfish), and anthozoa (sea anemone). Coelenterates are responsible for more envenomations or injections of venom than any other marine phylum.

Jellyfish are marine invertebrates having a bell-shaped, jelly-like substance enclosing its internal structure.

Most species of jellyfish have tentacles with stinging cells. The stinging cells may stun or kill other ocean life. Most jellyfish use stinging to either stunning prey before catching the prey or to defend themselves. Most jellyfish are not very dangerous to humans, but a few are highly toxic such as the box jelly fish in the Pacific Ocean along the Australian east coast and along the costs of Hawaii.

It is known to use different liquids for relieving the pain caused by the sting, subsequently to remove the stingers from the skin by scraping off the stingers from the skin, and finally to use antihistamines for soothing any final skin irritation.

Bees are flying insects closely related to wasps and ants. There are nearly 20,000 known species of bee, in nine recognized families. They are ubiquitous, present in every habitat on the planet that contains insect-pollinated flowering plants.

A bee that is away from the hive foraging for nectar or pollen will rarely sting. Except when stepped on or roughly handled. However, bees will actively seek out and sting when they perceive the hive to be threatened, often being alerted to this by the release of attack pheromones from other bees. The sting of a bee is barbed so that it lodges in the victim's skin, tearing loose from the bee's abdomen and leading to its death in minutes.

The main component of bee venom (apitoxin) responsible for pain in vertebrates is the toxin melittin, but histamine and other biogenic amines also contribute to pain and especially itching. Histamine is also released from body cells because of the reaction to the sting in allergic persons.

Apitoxin is acidic, and because of that it is often recommended to neutralize the venom. However, neutralizing a sting is unlikely to be effective as the venom is injected under the skin and deep into the tissues, where a topically applied alkali is unable to reach. Medications such as antipruritics, known as anti-itch drugs, are also used in locally treating an insect sting. When being stung by a bee, the immediate action is to scrape out the sting to avoid further venom to enter the body.

All the liquids and remedies for the above mentioned procedures are not always at hand where needed, namely at the beach or in the forest, and all liquids are even not always at hand at home.

US2007/0280926 A1 describes a sting apparatus and method of use. The apparatus is a portable kit that can be easily used to immediately treat jelly fish stings or stings from other stinging organisms such as bees, wasps, hornets, etc. The kit includes a solution including a proteinase toxin neutralizer to neutralize jelly fish stinger toxin proteins and tools coated with proteolytic agents to attract, attach and further neutralize jelly fish stingers.

### DISCLOSURE OF THE INVENTION

In order to ensure fast, correct and satisfactory treatment of persons having been stung by stinging organisms such as coelenterates, stinging nettles, or bees, it would be an advantage to have the agents and remedies for treating the stings combined in a single device of relatively small size to carry around at all times. It may be an object of the invention to overcome the disadvantages of not having the agents and remedies for the above mentioned procedures at hand where needed, namely at the beach or in the forest, and all agents are even not always at hand at home.

The treatment could also be used for animals such as pets, zoo animals or wild animals that may have become stung by stinging organisms such as coelenterates, stinging nettles, or bees. The object of the invention may also be to decrease the risk of being ill or even die from being stung, and to enhance people to take necessary precautions.

This object may be obtained by a device being adapted for keeping in a wallet, a pocket, a bag, or similar place portable to a person, said device being designed as a credit card in size with a length of approximately 85 mm, a width of approximately 54 mm, and a thickness of maximum 5 mm, and said card being made of a relatively stiff material, such as cardboard, wood, metal or plastic, said card having at least one edge being sharpened in relation to the remaining part of the card, and said card having at least one recess or location for holding at least one agent for applying on the skin of a person or an animal.

In another embodiment, the object may be a device for use in treating sting by a stinging organism, said treatment being performed by removing stingers from the skin, and said treatment also being performed by applying at least one agent on the skin of a person or an animal for relieving pain and/or for soothing the skin, said device being adapted for keeping in a wallet, a pocket, a bag, or similar place portable to a person, said device being designed substantially as a credit card in size with a length of between 75 mm and 85 mm, possibly approximately 83 mm, a width of between 45 mm and 55 mm, possibly approximately 54 mm, and a thickness of maximum 5 mm, and said card preferably being made of a relatively stiff material, such as cardboard, metal, wood or plastic, said card having at least one edge being sharpened in relation to a remaining part of the card, and said card having at least one recess or location for holding at least one agent for applying on the skin of a person or an animal.

As mentioned, the device according to the invention has a size corresponding to a credit card, i.e. with a length of about 85 mm, a width of about 54 mm and a total thickness less than 5 mm, preferably less than 2 mm.

In another embodiment, the device according to the invention has a design as a credit card in size with a length of between 80 mm and 90 mm, a width of between 49 mm and 59 mm, and a thickness of between 0.25 mm and 4.00 mm.

In yet another embodiment, the device according to the invention is designed substantially as a credit card in size, has a length of between 80 mm and 85 mm, a width of between 45 mm and 55 mm, and a thickness of between 0.25 mm and 3 mm.

Alternatively, the device according to the invention is designed substantially as a credit card in size, has a length of between 80 mm and 85 mm, a width of between 50 mm and 55 mm, and a thickness of between 0.25 mm and 3 mm.

In one embodiment, a device according to the invention where said edge being sharpened in relation to a remaining part of the card has a length of between 0.25 mm and 10 mm, and an angle of inclination in relation to an extension of a plane of the card of between 15 degrees and 75 degrees.

In another embodiment, a device according to the invention where said edge being sharpened in relation to a remaining part of the card has a length of between 2 mm and 6 mm, and an angle of inclination in relation to an extension of a plane of the card of between 15 degrees and 45 degrees.

When being stung by a bee, the immediate action is to scrape out the sting to avoid further venom to enter the body.

In one embodiment, a device according to the invention is made so that the card is provided with a slit adapted for use in releasing a bee sting, said slit having an orifice at one edge of the device, preferably having an orifice at one of the at least one sharpened edge of the device.

In another embodiment, a device according to the invention is made so that the card is provided with a slit adapted for use in releasing a bee sting, said slit having an orifice at a corner of the device, preferably having an orifice at a corner positioned along the at least one sharpened edge of the device.

In yet another embodiment, a device according to the invention is made so that the slit adapted for use in releasing a bee sting has a tapering configuration with a wide end of the tapering configuration constituting the orifice and with the narrow end of the tapering configuration being opposite the orifice of the slit.

The immediate treatment of the sting involves relieving the pain and removing the stingers. Regarding stings from coelenterates, prior to removing the stingers, an immediate treatment of the coelenterates' stingers may be performed to inhibit further venom discharge. A subsequent treatment of the sting involves soothing the skin for long-term reducing of pain and itch. Removing the stingers is very important; relieving the pain and itch may be done, but need not be done. Soothing the skin may be done, but need not be done.

The device according to the invention may be designed so that a folded first tissue containing a pain relieving agent has a length and a width of about 44 mm x 44 mm, and that the device has a second tissue having a length and a width of about 24 x 44 mm, said second tissue containing an antipruritic agent or a skin soothing agent or a stinger neutralization agent. In another embodiment, the device according to the invention may be designed so that a folded first tissue containing a stinger neutralization agent has a length and a width of about 44 mm x 44 mm, and that the device has a second tissue having a length and a width of about 24 x 44 mm, said second tissue containing an antipruritic agent or a skin soothing agent or a pain relieving agent.

Alternatively, the folded first tissue may have a length and a width of about 56 x 44 mm, whereas the second folded tissue may correspondingly have a length and a width of about 12 x 44 mm, or vice versa, so that the first tissue may be larger than second tissue, or vice versa.

Alternatively, the device according to the invention may be designed so that that a folded first tissue containing a pain relieving agent has a length and a width of about 44 mm x 44 mm, and that the device has a second and a third tissue both having a length and a width of about 12 x 44 mm, said second tissue containing an antipruritic agent or a skin soothing agent, said third tissue containing an antipruritic agent or a skin soothing agent.

Alternatively, the device according to the invention may be designed so that that a folded first tissue containing a stinger neutralization agent has a length and a width of about 44 mm x 44 mm, and that the device has a second and a third tissue both having a length and a width of about 12 x 44 mm, said second tissue containing an antipruritic agent or a skin soothing agent or a pain relieving agent, said third tissue containing an antipruritic agent or a skin soothing agent or a pain relieving agent. Alternatively to having folded tissues with pain relieving and/or skin soothing and/or antipruritic and/or stinger neutralization agents, the card may have recesses or just have designated locations for holding a paste or a flat solid brick of pain relieving agent, and/or antipruritic agent, and/or stinger neutralization agent, and/or skin soothing agent.

The various forms of administration may solve different problems, e.g. if a victim has a large area affected by stingers a large amount or volume of agent might be necessary. This could be solved by the card holding a solid brick of concentrated agent for dilution in water.

Alternatively to having both pain relieving agent and skin soothing agent, the card may have only pain relieving agent, provided on a tissue or as a paste or a solid brick or as foil-wrapped liquid, or the card may have only skin soothing agent, provided on a tissue or as a paste or a solid brick.

Alternatively to having several agents, the card may have only one agent, provided on a tissue or as foil-wrapped liquid or as a paste or a solid brick.

In still alternative embodiments, more than one recess or two recesses or locations may be provided. The device is thus capable of holding more than one or two agents, or the device may hold more amounts of the one or of the two agents, possibly for different doses of one or both agents being applied within intervals during treatment.

Preferably, the device according to the invention is designed so that any tissue holding either the pain relieving agent or the skin soothing agent has a size which is considerably greater that the size of reservoir or the location where the tissue is intended for being positioned on the surface of the card.

Preferably, the device according to the invention is designed so that any tissue holding any type of the before mentioned agents has a size which is considerably greater that the size of recess or the location where the tissue is intended for being positioned on the surface of the card. The tissue is then folded in order for the larger sized tissue to fit the at least one recess or location of the card.

In one embodiment, the device according to the invention comprises at least one agent being, said agent being provided on a tissue or as foil-wrapped liquid or as a paste or a solid brick and being located in the at least one recess or location of the card. Depending on the kind of agent and depending on the need of the agent for immediate use, different means of properties, i.e. impregnated in a tissue, applicable as a paste, or to be dissolved as a solid brick in liquid may be used for the card to hold the agent during production, packaging and storing of the device and during subsequent carrying along of the device by the end user.

In another embodiment, the device according to the invention comprises at least two different agents, said agents being provided on a tissue or as foil-wrapped liquid or as a paste or a solid brick and being located in the at least one recess of the card, possibly being located in two different recesses of the card.

Providing different agents may be useful, either for providing the possibility of soothing pain and/or for curing immediate skin disorder of persons being stung by one or another different organism. Providing different agents may also be useful for providing the possibility of soothing pain and curing immediate skin disorder or other differentiated treatment of person being stung by one organism. The different agents may in one embodiment be provided in two different recesses, said recesses resulting in the overall thickness of the card being minimised.

In yet another embodiment, a device according to the invention comprises at least two different agents, said agents being provided on a tissue or as foil-wrapped liquid or as a paste or a solid brick and being located in the at least one location of the card, possibly being located in two different locations of the card.

Providing different agents may be useful, either for providing the possibility of soothing pain and/or for curing immediate skin disorder of persons being stung by one or another different organism. Providing different agents may also be useful for providing the possibility of soothing pain and curing immediate skin disorder or other differentiated treatment of person being stung by one organism. The different agents may in one embodiment be provided in two different locations, said location resulting in the overall stiffness of the card being maximised

A card may be made more stiff only having locations and not recesses, such card only having locations not having a smaller thickness, and such card only having locations not having a reduced stiffness, where recesses would be located.

Alternatively, a device according to the invention comprises at least two different agents being provided on a tissue or as foil-wrapped liquid or as a paste or a solid brick and each of said at least two different agents being covered by means of differently coloured films, or being of different colour or being provided with or made with other marking for facilitating to discern between the at least two different agents of the device. By being able to differentiate between the different agent, correct and fast treatment of the person to be stung is ensured.

In one embodiment the device according to the invention is intended for being used in treating a sting by a member of the coelenterates, and said at least one recess or location holds at least antivenin as agent for applying on the skin of a person or an animal.

In another embodiment, the device according to the invention is intended for being used in treating a sting by a jellyfish, and said at least one recess or location holds at least one of the following agents: Diluted acetic acid, ammonia, or citrus juice for medical treatment of the person having been stung.

In yet another embodiment, the device according to the invention is intended for being used in treating a sting by a sea nettle, and where said at least one recess or location holds at least baking soda for medical treatment of the person having been stung.

Sea anemones and some kinds of coral also contain nematocysts, and stings from anemones or coral are similar to a jellyfish sting. Sea anemones are small creatures that attach themselves to rocks, coral, or even the backs of crabs. They use their flower-like tentacles to catch small marine animals, which are paralyzed by the anemone's venom.

In a further embodiment, the device according to the invention is intended for being used in treating a sting by a sea anemone, and said at least one recess or location holds at least white vinegar for medical treatment of the person having been stung.

In yet a further embodiment, the device according to the invention is intended for being used in treating a sting by a bee, and said at least one recess or location holds at least diphenhydramine for medical treatment of the person having been stung.

In still a further embodiment, the device according to the invention is intended for being used in treating a sting by a stinging nettle, and said at least one recess or location holds at least aluminium acetate for medical treatment of the person having been stung.

The different agents mentioned above in relation to the different organisms are agents which are well-suited for immediate treatment of a person being stung. Other agents than the ones mentioned will also be possible to provide within the general common knowledge of the person skilled in the art. However, it is preferred that the one or more agents provided are suited for immediate treatment and not are intended for long term treatment only, e.g. antibiotics. Immediate treatment is meant as treatment at site of the person being stung, or within a period of maximum 12 hours after the person have been stung.

In a preferred embodiment, the device according to the invention has at least one edge being sharpened in relation to a remaining part of the card has a length of between 3 mm and 4 mm, and an angle of inclination 10 in relation to an extension of a plane of the card of between 25 degrees and 30 degrees. By providing the at least one edge with a sharpening as mentioned, a proper but still not painful scraping is obtained of the skin of the person being treated.

With the purpose of facilitating to discern between the individual parts of the device, the device may suitably be designed so that the different agents are covered by means of films with mutually different colour or other marking. Suitably, the device is further designed so that the cover films are provided with a corner or tongue section, which is not connected with the device, and which is intended for use in pulling off the cover films.

The device according to the invention is made of a relatively stiff material, e. g. cardboard or metal or wood or plastic for ensuring a proper scraping function of the card. The choice of material depends on the intended use of the card, i.e. which organism a person being stung is intended for being treated for, and depends on the maximum price and/or the intended size of the device, especially the intended thickness of the device, and especially the intended thickness of the card, apart from the agents, of the device.

### DETAILED DESCRIPTION OF THE INVENTION

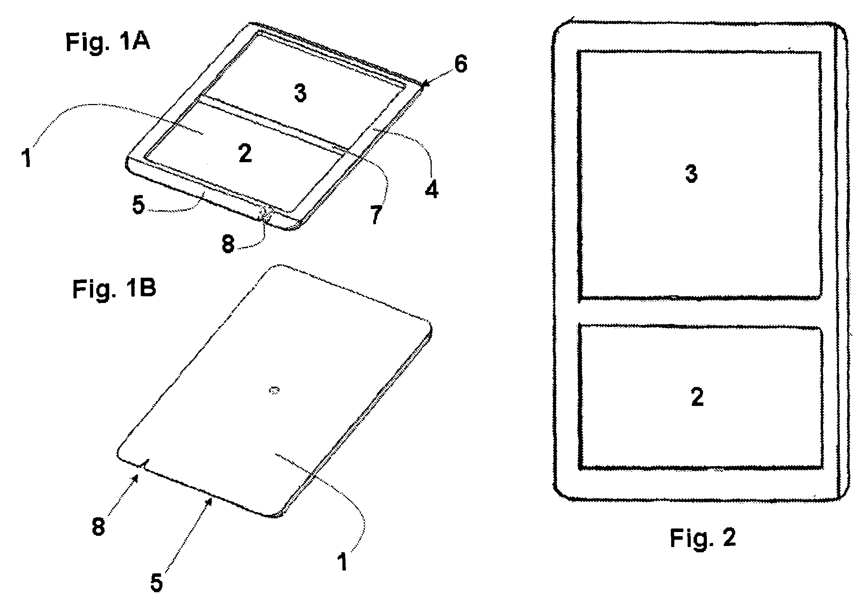

Fig. 1 shows a card 1 having a selected dimension corresponding to the size of a credit card. The card thus have a length of approximately 85 mm, preferably a length of 83 mm, a width of approximately 54 mm and a thickness of maximum 5 mm, preferably a thickness of maximum 3 mm such as approximately 2 mm. In the embodiment shown, all corners 6 of the card are rounded. Alternatively, all or some of the corners 6 of the card may be pointed or rounded with another radius than the one shown.

The card is provided with a first recess 2 and a second recess 3 provided in the surface 4 of the card and divided by a division bulge 7. The recesses 2,3 are intended for holding different agents for immediate relieving pain and for subsequent soothing of the skin. The recesses 2, 3 are shown to be of the same size. The recesses may however be of different size as seen in figure 2, or the first recess may be larger than the second recess. Depending on the agent used for relieving the pain and the agent used for soothing the skin, possibly the one agent needs to be applied in a larger amount than the other agent, or vice versa.

Alternatively, one or both of the recesses are omitted, and the areas of the card where the recesses are illustrated may be used as selected locations of the surface 4 of the card for the agents for relieving the pain and for soothing the skin, however, without recesses as such provided in the surface of the card for holding the agents. The agents may be provided contained in a tissue, as a foil-wrapped liquid, or applied as a paste or provided as a small solid brick.

The first recess 2 or location of the card is intended for holding an agent for neutralizing the stingers and relieving the pain as part of an immediate treatment after having been stung by a stinging organism such as a jellyfish. The agent for neutralizing the stingers and relieving the pain may be provided as a solid brick of concentrated agent for dilution in water provided in the recess 2 or he location, or as a foil-wrapped liquid provided in the recess 2 or location, or the agent for neutralizing the stinger and relieving the pain may be provided as a paste filled into the recess 2, or contained in a tissue folded to fit the recess 2.

The agent for neutralizing the stinger and relieving the pain as part of an immediate treatment may be one or more of any of the following substances, either provided as one of the substances only or provided as a selected mixture of more substances: A 5% to 25% acetic acid solution such as vinegar, ammonia, or citrus juice, meat tenderizer, sodium bicarbonate such as baking soda, diphenhydramine, aluminium acetate, a deactivating solution or sea water.

The second recess 3 or location of the card is holding an agent for soothing the skin after having performed the immediate treatment including neutralizing the stinger and relieving the pain. The agent for soothing the skin may be provided on a tissue folded to fit the recess or the location, or as a foil-wrapped liquid provided in the recess 2 or he location, or the agent for soothing the skin may be provided as a paste filled into the recess or as a small brick.

The agent for soothing the skin as part of a subsequent treatment may be one or more of any of the following substances, either provided as one of the substances only or provided as a selected mixture of more substances: antihistamines containing diphen-hydramine such as Benadryl™, hydrocortisone cream, antivenom comprising antibodies against the venom's active molecule, any anaesthetic agent or Papaine enzyme.

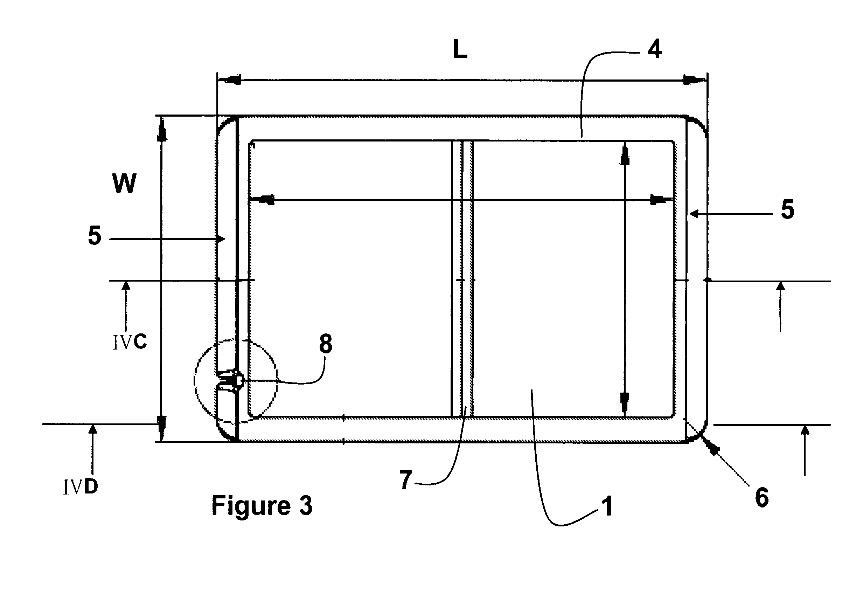

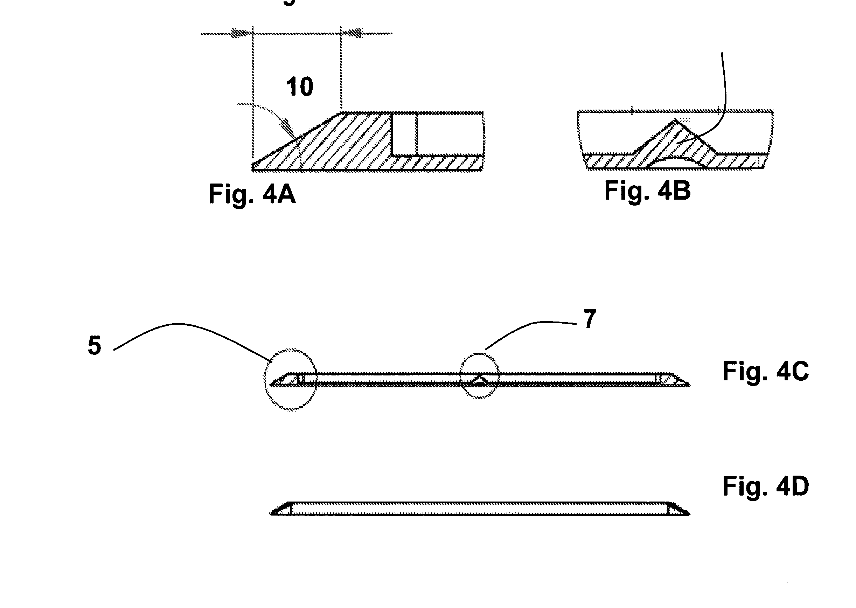

In another preferred embodiment, a device according to the invention is made so that the card is provided with a slit 8 (see Fig. 3) adapted for use in releasing a bee sting, said slit having a tapering configuration with a wide end of the tapering configuration constituting the orifice and with the narrow end of the tapering configuration being opposite the orifice of the slit. The card is preferably provided with one or two sharpened edges 5 along the length of the card and/or along the width of the card. Alternatively the card is provided with three or four sharpened edges along the length of the card and along the width of the card. In the embodiments shown in figure 1, the card is provided with two sharp edges 5 along the width of the card. In the embodiments shown in figure 2, the card is only provided with one sharp edge 5 along the length of the card.

The one or more sharpened edges are provided by the provision of the one or both edges having a smaller thickness than the overall and/or maximum thickness of the card, such as the thickness immediate outside the edges. One or two or even more sharpened edges make the card more suited for scraping off the stingers from the skin The one or more sharpened edges may be passed along the skin with the sharpened edge leading, so that the stingers are scraped up onto the card and off the skin.

The one or more sharpened edges, if provided on the card, have a thickness of less than 75% of the overall thickness of the card, preferably a thickness of less than 50% of the overall thickness of the card. The sharper the one or more edges are, the better the one or more edges are for scraping off the stingers. It is important that the stingers are scraped off by the edge "shovelling" the stingers by means of the card being pushed along the skin with the sharpened edge along the skin as the leading edge of the card.

Different edges of the card may have different sharpness. This may give the advantage of one edge being for scraping on the skin of a child having a more sensitive skin and another edge being for scraping on the skin of an older person having a less sensitive skin towards scraping of the card along the skin. Also, it may have the advantage of being able of scraping with different sharpened edges more than once along the skin.

In the above, the invention is describes with reference to the accompanying drawing and with reference to specific embodiments. The person skilled in art may come up with other embodiments which all lie within the scope of protection as defined by the claims.

## Claims

1. A device for use in treating sting by a stinging organism, said treatment being performed by removing stingers from the skin, and said treatment also being performed by applying at least one agent on the skin of a person or an animal for relieving pain and/or for soothing the skin, said device being adapted for keeping in a wallet, a pocket, a bag, or similar place portable to a person, said device being designed substantially as a credit card in size with a length of between 75 mm and 85 mm, possibly approximately 83 mm, a width of between 45 mm and 55 mm, possibly approximately 54 mm, and a thickness of maximum 5 mm, and said card having at least one edge being sharpened in relation to a remaining part of the card, and said card having at least one recess or location for holding at least one agent for applying on the skin of a person or an animal.

2. A device for use according to claim 1, where the device is intended for being used in treating a sting by a member of the coelenterates, and where said at least one recess or location holds at least antivenin as agent for applying on the skin of a person or an animal.

3. A device for use according to claim 1, where the device is intended for being used in treating a sting by a jellyfish, and where said at least one recess or location holds at least one of the following agents: Diluted acetic acid, ammonia, or citrus juice for medical treatment of the person having been stung.

4. A device for use according to claim 1, where the device is intended for being used in treating a sting by a sea nettle, and where said at least one recess or location holds at least baking soda for medical treatment of the person having been stung.

5. A device for use according to claim 1, where the device is intended for being used in treating a sting by a sea anemone, and where said at least one recess or location holds at least white vinegar for medical treatment of the person having been stung.

6. A device for use according to claim 1, where the device is intended for being used in treating a sting by a bee, and where said at least one recess or location holds at least diphenhydramine for medical treatment of the person having been stung.

7. A device for use according to claim 1, where the device is intended for being used in treating a sting by a stinging nettle, and where said at least one recess or location holds at least aluminium acetate for medical treatment of the person having been stung.

8. A device according to any of the preceding claims 1-7, where said device being designed substantially as a credit card in size has a length of between 80 and 85 mm, a width of between 45 and 55 mm, and a thickness of between 0.25 and 3 mm.

9. A device according to any of the preceding claims 1-7, where said device being designed as a credit card in size with a length of between 80-85 mm, a width of between 50-55 mm, and a thickness of between 0.25 and 3 mm.

10. A device according to any of the preceding claims 1-9, where said edge being sharpened in relation to a remaining part of the card has a length of between 0.25-10 mm, and an angle of inclination in relation to an extension of a plane of the card of between 15 and 75 degrees.

11. A device according to any of the preceding claims 1-9, where said edge being sharpened in relation to a remaining part of the card has a length of between 2 and 6 mm, and an angle of inclination in relation to a planar extension of the card of between 15 and 45 degrees.

12. A device according to any of the preceding claims 1-11, wherein said card is provided with a slit adapted for use in releasing a bee sting, said slit having an orifice at one edge of the device, preferably having an orifice at one of the at least one sharpened edge of the device.

13. A device according to any of the preceding claims 1-11, wherein said card is provided with a slit adapted for use in releasing a bee sting, said slit having an orifice at a corner of the device, preferably having an orifice at a corner positioned along the at least one sharpened edge of the device.

14. A device according to claims 12-13, wherein said slit adapted for use in releasing a bee sting has a tapering configuration with a wide end of the tapering configuration constituting the orifice and with the narrow end of the tapering configuration being opposite the orifice of the slit.

15. A device according to any of the preceding claims 1-14, where the device comprises at least one agent being provided on a tissue or as foil-wrapped liquid or as a paste or a solid brick and being located in the at least one recess or location of the card.

16. A device according to any of the preceding claims 1-14, where the device comprises at least two different agents being provided on a tissue or as foil-wrapped liquid or as a paste or a solid brick and being located in the at least one recess of the card, possibly being located in two different recesses of the card.

17. A device according to any of the preceding claims 1-14, where the device comprises at least two different agents being provided on a tissue or as foil-wrapped liquid or as a paste or a solid brick and being located in the at least one location of the card, possibly being located in two different locations of the card.

18. A device according to any of the preceding claims 1-17, where the device comprises at least two different agents being provided on a tissue or as foil-wrapped liquid or as a paste or a solid brick, and each of said at least two different agents being covered by means of differently coloured films, or being of different colour or being provided with or made with other marking for facilitating to discern between the at least two agents of the device.

## Patentansprüche

1. Vorrichtung zur Verwendung beim Behandeln eines Stichs durch einen stechenden Organismus, wobei die Behandlung ausgeführt wird, indem Stacheln aus der Haut entfernt werden und die Behandlung ebenfalls ausgeführt wird, indem mindestens ein Mittel auf die Haut einer Person oder eines Tiers zum Lindern von Schmerz und/oder zum Beruhigen der Haut aufgetragen wird, wobei die Vorrichtung angepasst ist, um in einer Brieftasche, einer Tasche, einem Beutel oder einer ähnlichen Stelle, welche ein Mensch mit sich führen kann, aufbewahrt zu werden, wobei die Vorrichtung im Wesentlichen hinsichtlich der Größe wie eine Kreditkarte mit einer Länge zwischen 75 mm und 85 mm, möglicherweise ungefähr 83 mm, einer Breite zwischen 45 mm und 55 mm, möglicherweise ungefähr 54 mm, und einer Dicke von maximal 5 mm gestaltet ist und wobei die Karte mindestens eine Kante aufweist, die in Bezug auf einen übrigen Teil der Karte zugespitzt ist, und die Karte mindestens eine Ausnehmung oder Stelle zum Aufnehmen von mindestens einem Mittel zum Auftragen auf die Haut einer Person oder eines Tiers aufweist.

2. Vorrichtung zur Verwendung nach Anspruch 1, wobei die Vorrichtung dazu bestimmt ist, beim Behandeln eines Stichs durch ein Mitglied der Coelenterata verwendet zu werden, und wobei die mindestens eine Ausnehmung oder Stelle mindestens ein Gegengift als Mittel zum Auftragen auf die Haut einer Person oder eines Tiers beinhaltet.

3. Vorrichtung zur Verwendung nach Anspruch 1, wobei die Vorrichtung dazu bestimmt ist, beim Behandeln eines Stichs durch eine Qualle verwendet zu werden, und wobei die mindestens eine Ausnehmung oder Stelle mindestens eines der folgenden Mittel beinhaltet: Verdünnte Essigsäure, Ammoniak oder Zitrusfruchtsaft, zur medizinischen Behandlung der Person, die gestochen worden ist.

4. Vorrichtung zur Verwendung nach Anspruch 1, wobei die Vorrichtung dazu bestimmt ist, beim Behandeln eines Stichs durch eine Seenessel verwendet zu werden, und wobei die mindestens eine Ausnehmung oder Stelle mindestens Natriumhydrogencarbonat zur medizinischen Behandlung der Person, die gestochen worden ist, beinhaltet.

5. Vorrichtung zur Verwendung nach Anspruch 1, wobei die Vorrichtung dazu bestimmt ist, beim Behandeln eines Stichs durch eine Seeanemone verwendet zu werden, und wobei die mindestens eine Ausnehmung oder Stelle mindestens weißen Essig zur medizinischen Behandlung der Person, die gestochen worden ist, beinhaltet.

6. Vorrichtung zur Verwendung nach Anspruch 1, wobei die Vorrichtung dazu bestimmt ist, beim Behandeln eines Stichs durch eine Biene verwendet zu werden, und wobei die mindestens eine Ausnehmung oder Stelle mindestens Diphenhydramin zur medizinischen Behandlung der Person, die gestochen worden ist, beinhaltet.

7. Vorrichtung zur Verwendung nach Anspruch 1, wobei die Vorrichtung dazu bestimmt ist, beim Behandeln eines Stichs durch eine Brennnessel verwendet zu werden, und wobei die mindestens eine Ausnehmung oder Stelle mindestens Aluminiumacetat zur medizinischen Behandlung der Person, die gestochen worden ist, beinhaltet.

8. Vorrichtung nach einem der vorangegangenen Ansprüche 1-7, wobei die Vorrichtung, die hinsichtlich der Größe im Wesentlichen wie eine Kreditkarte gestaltet ist, eine Länge zwischen 80 und 85 mm, eine Breite zwischen 45 und 55 mm und eine Dicke zwischen 0,25 und 3 mm aufweist.

9. Vorrichtung nach einem der vorangegangenen Ansprüche 1-7, wobei die Vorrichtung hinsichtlich der Größe wie eine Kreditkarte mit einer Länge zwischen 80 und 85 mm, einer Breite zwischen 50 und 55 mm und einer Dicke zwischen 0,25 und 3 mm gestaltet ist.

10. Vorrichtung nach einem der vorangegangenen Ansprüche 1-9, wobei die Kante, die in Bezug auf einen übrigen Teil der Karte zugespitzt ist, eine Länge zwischen 0,25 und 10 mm und einen Neigungswinkel in Bezug auf eine Verlängerung einer Ebene der Karte zwischen 15 und 75 Grad aufweist.

11. Vorrichtung nach einem der vorangegangenen Ansprüche 1-9, wobei die Kante, die in Bezug auf einen übrigen Teil der Karte zugespitzt ist, eine Länge zwischen 2 und 6 mm und einen Neigungswinkel in Bezug auf eine ebene Verlängerung der Karte zwischen 15 und 45 Grad aufweist.

12. Vorrichtung nach einem der vorangegangenen Ansprüche 1-11, wobei die Karte mit einem Schlitz, der für eine Verwendung beim Herauslösen eines Bienenstichs angepasst ist, ausgestattet ist, wobei der Schlitz eine Öffnung an einer Kante der Vorrichtung aufweist, wobei sie vorzugsweise eine Öffnung an einer der mindestens einen zugespitzten Kante der Vorrichtung aufweist.

13. Vorrichtung nach einem der vorangegangenen Ansprüche 1-11, wobei die Karte mit einem Schlitz, der für eine Verwendung beim Herauslösen eines Bienenstichs angepasst ist, ausgestattet ist, wobei der Schlitz eine Öffnung an einer Ecke der Vorrichtung aufweist, wobei sie vorzugsweise eine Öffnung an einer Ecke, die sich entlang der mindestens einen zugespitzten Kante der Vorrichtung befindet, aufweist

14. Vorrichtung nach den Ansprüchen 12-13, wobei der Schlitz, der für eine Verwendung beim Herauslösen eines Bienenstichs angepasst ist, eine sich verjüngende Konfiguration aufweist, wobei ein breites Ende der sich verjüngenden Konfiguration die Öffnung bildet und wobei das enge Ende der sich verjüngenden Konfiguration der Öffnung des Schlitzes gegenüber liegt.

15. Vorrichtung nach einem der vorangegangenen Ansprüche 1-14, wobei die Vorrichtung mindestens ein Mittel umfasst, welches auf einem Gewebe oder als eine mit Folie umhüllte Flüssigkeit oder als eine Paste oder ein fester Ziegel bereitgestellt ist und welches sich in der mindestens einen Ausnehmung oder Stelle der Karte befindet.

16. Vorrichtung nach einem der vorangegangenen Ansprüche 1-14, wobei die Vorrichtung mindestens zwei verschiedene Mittel umfasst, die auf einem Gewebe oder als eine mit Folie umhüllte Flüssigkeit oder als eine Paste oder ein fester Ziegel bereitgestellt sind, und welche sich in der mindestens einen Ausnehmung der Karte befinden, wobei sie sich gegebenenfalls in zwei unterschiedlichen Ausnehmungen der Karte befinden.

17. Vorrichtung nach einem der vorangegangenen Ansprüche 1-14, wobei die Vorrichtung mindestens zwei verschiedene Mittel umfasst, die auf einem Gewebe oder als eine mit Folie umhüllte Flüssigkeit oder als eine Paste oder ein fester Ziegel bereitgestellt sind, und welche sich in der mindestens einen Stelle der Karte befinden, wobei sie sich gegebenenfalls in zwei unterschiedlichen Stellen der Karte befinden.

18. Vorrichtung nach einem der vorangegangenen Ansprüche 1-17, wobei die Vorrichtung mindestens zwei verschiedene Mittel umfasst, die auf einem Gewebe oder als eine mit Folie umhüllte Flüssigkeit oder als eine Paste oder ein fester Ziegel bereitgestellt sind, und wobei jedes der mindestens zwei verschiedenen Mittel mittels unterschiedlich gefärbter Folien bedeckt ist oder eine unterschiedliche Farbe aufweist oder mit einer anderen Markierung zur Vereinfachung der Unterscheidung zwischen den mindestens zwei Mitteln der Vorrichtung versehen oder hergestellt ist.

## Revendications

1. Dispositif pour utilisation dans le traitement de piqûres provoquées par des organismes piqueurs, ledit traitement étant réalisé en éliminant les dards de la peau, et ledit traitement étant également réalisé en appliquant au moins un agent sur la peau d'une personne ou d'un animal afin de soulager la douleur et/ou d'apaiser la peau, ledit dispositif étant adapté pour être contenu dans un portefeuille, une poche, un sac ou un endroit similaire, portable par une personne, ledit dispositif étant conçu sensiblement de la taille d'une carte de crédit, avec une longueur comprise entre 75 mm et 85 mm, éventuellement approximativement 83 mm, une largeur comprise entre environ 45 mm et 55 mm, éventuellement approximativement 54 mm et une épaisseur de 5 mm maximum, et ladite carte ayant au moins un bord acéré en relation avec une partie restante de la carte, ladite carte ayant au moins une cavité ou un emplacement pour contenir au moins un agent à appliquer sur la peau d'une personne ou d'un animal.

2. Dispositif pour utilisation selon la revendication 1, dans lequel le dispositif est destiné à être utilisé dans le traitement d'une piqûre d'un membre des coelentérés, et dans lequel ladite au moins une cavité ou un emplacement contient au moins un antivenin comme agent à appliquer sur la peau d'une personne ou d'un animal.

3. Dispositif pour utilisation selon la revendication 1, dans lequel le dispositif est destiné à être utilisé dans le traitement d'une piqûre d'une méduse, et dans lequel ladite au moins une cavité ou un emplacement contient au moins un des agents suivants : acide acétique dilué, ammoniaque, ou jus de citron pour le traitement médical de la personne ayant été piquée.

4. Dispositif pour utilisation selon la revendication 1, dans lequel le dispositif est destiné à être utilisé dans le traitement d'une piqûre par une ortie de mer, et dans lequel ladite au moins cavité ou un emplacement contient au moins du bicarbonate de soude, pour le traitement médical de la personne ayant été piquée.

5. Dispositif pour utilisation selon la revendication 1, dans lequel le dispositif est destiné à être utilisé dans le traitement d'une piqûre par une anémone de mer, et dans lequel ladite au moins une cavité ou un emplacement contient au moins du vinaigre blanc pour le traitement médical de la personne ayant été piquée.

6. Dispositif pour utilisation selon la revendication 1, dans lequel le dispositif est destiné à être utilisé dans le traitement d'une piqûre par une abeille, et dans lequel ladite au moins une cavité ou un emplacement contient au moins de la diphénhydramine pour le traitement médical de la personne ayant été piquée.

7. Dispositif pour utilisation selon la revendication 1, dans lequel le dispositif est destiné à être utilisé dans le traitement d'une piqûre par une ortie dioïde, et dans lequel ladite au moins une cavité ou un emplacement contient au moins de l'acétate d'aluminium pour le traitement médical de la personne ayant été piquée.

8. Dispositif selon l'une quelconque des revendications précédentes 1 à 7, dans lequel ledit dispositif conçu sensiblement de la taille d'une carte de crédit, a une longueur comprise entre 80 et 85 mm, une largeur comprise entre 45 et 55 mm, et une épaisseur comprise entre 0,25 et 3 mm.

9. Dispositif selon l'une quelconque des revendications précédentes 1 à 7, dans lequel ledit dispositif conçu de la taille d'une carte de crédit, a une longueur comprise entre 80 et 85 mm, une largeur comprise entre 50 et 55 mm, et une épaisseur comprise entre 0,25 et 3 mm.

10. Dispositif selon l'une quelconque des revendications précédentes 1 à 9, dans lequel ledit bord acéré en relation avec une partie restante de la carte a une longueur comprise entre 0,25 et 10 mm, et un angle d'inclinaison en relation avec une extension d'un plan de la carte entre 15 et 75 degrés.

11. Dispositif selon l'une quelconque des revendications précédentes 1 à 9, dans lequel ledit bord acéré en relation avec une partie restante de la carte a une longueur comprise entre 2 et 6 mm, et un angle d'inclinaison en relation avec une extension d'un plan de la carte entre 15 et 45 degrés.

12. Dispositif selon l'une quelconque des revendications précédentes 1 à 11, dans lequel ladite carte est dotée d'une fente, adaptée pour être utilisée pour enlever une piqûre d'abeille, ladite fente ayant un orifice sur un bord du dispositif, de préférence ayant un orifice sur l'un desdits au moins un bord acéré du dispositif.

13. Dispositif selon l'une quelconque des revendications précédentes 1 à 11, dans lequel ladite carte est dotée d'une fente adaptée pour être utilisée pour enlever une piqûre d'abeille, ladite fente ayant un orifice à un coin du dispositif, ayant de préférence un orifice à un coin, positionné le long dudit au moins un bord acéré du dispositif.

14. Dispositif selon les revendications 12 à 13, dans lequel ladite fente adaptée pour une utilisation dans l'enlèvement d'une piqûre d'abeille a une configuration conique, l'extrémité large de la configuration conique constituant l'orifice et l'extrémité étroite de la configuration conique étant à l'opposé de l'orifice de la fente.

15. Dispositif selon l'une quelconque des revendications 1 à 14, dans lequel le dispositif comprend au moins un agent fourni sur un tissu ou un liquide enveloppé dans une feuille ou sous la forme d'une pâte ou d'une brique solide et situé dans ladite au moins une cavité ou un emplacement de la carte.

16. Dispositif selon l'une quelconque des revendications précédentes 1 à 14, dans lequel le dispositif comprend au moins deux agents différents fournis sur un tissu ou un liquide enveloppé dans une feuille ou sous la forme d'une pâte ou brique solide et situés dans ladite au moins une cavité de la carte, éventuellement situés dans deux cavités différentes de la carte.

17. Dispositif selon l'une quelconque des revendications précédentes 1 à 14, dans lequel le dispositif comprend au moins deux agents différents fournis sur un tissu ou un liquide enveloppé dans une feuille ou sous la forme d'une pâte ou brique solide et situés dans ledit au moins un emplacement de la carte, éventuellement situés dans deux emplacements différents de la carte.

18. Dispositif selon l'une quelconque des revendications précédentes 1 à 17, dans lequel le dispositif comprend au moins deux agents différents fournis sur un tissu ou un liquide enveloppé dans une feuille ou sous la forme d'une pâte ou brique solide, et chacun desdits au moins deux agents différents étant recouvert au moyen de films de couleur différente, ou étant de couleur différente ou étant doté de ou réalisé avec un autre marquage, pour permettre de discerner plus facilement lesdits au moins deux agents du dispositif
